# EUROPÄISCHE PATENTSCHRIFT

(11) **EP 0 809 982 B1**
(45) Veröffentlichungstag und Bekanntmachung des Hinweises auf die Patenterteilung: **15.01.2003**
(21) Anmeldenummer: 97108088.2
(22) Anmeldetag: 17.05.1997
(51) Int. Cl.: A61F 2/18

(54) **Steigbügel-Prothese zur Implantation im Mittelohr**
Stapedial prosthesis for implantation in the middle ear
Prothèse d'étrier pour implantation dans l'oreille moyenne

(30) Priorität: 31.05.1996 DE 29609687 U
(43) Veröffentlichungstag der Anmeldung: 03.12.1997
(73) Patentinhaber: Heinz Kurz GmbH Medizintechnik, 72144 Dusslingen (DE)
(72) Erfinder: à Wengen, Daniel F., Dr., 4031 Basel (CH)
(74) Vertreter: Möbus, Rudolf, Dipl.-Ing.

(56) Entgegenhaltungen:
- EP-A- 0 563 767
- FR-A- 1 583 971
- SU-A- 175 178
- US-A- 3 711 869
- US-A- 3 722 003
- US-A- 5 370 689

## Beschreibung

Die Erfindung betrifft eine Steigbügel-Prothese zur Implantation im Mittelohr nach dem Oberbegriff des Anspruchs 1.

Zur Schallübertragung im Mittelohr bei defekten Gehörknöchelchen sind bereits mehrere Arten von Prothesen bekannt. Als Ersatz für den Steigbügel wird beispielsweise gemäß der EP 0 563 767 A eine Prothese verwendet, die aus einem dickeren kolbenförmigen Teil besteht, an dem sich ein schlankeres Schaftteil anschließt. Das Schaftende wird am langen Ambossfortsatz befestigt. Hierzu waren bislang am Schaftende starre Kopplungskörper zum Aufsatz auf den langen Ambossfortsatz in Gebrauch. Die bekannten Prothesen führten jedoch häufig zu Einschnürungen am langen Ambossfortsatz, wodurch Nekrosen auftreten können. Auch kann es durch Abschnüren von Versorgungsgefäßen zu einer Unterernährung des Processus lenticularis kommen. Eine Arosion des Processus lenticularis kann die Folge sein, wodurch auch der Halt der Prothese nicht mehr gewährleistet ist. Weitere Nachteile der bisherigen Prothesen bestanden darin, dass ihr Fixieren am langen Ambossfortsatz in der Stapedektomie höchste Ansprüche an den Operateur stellt.

Aus der US 3,711,869 A und der US 5,370,689 A sind Prothesen mit geschlitzten Ösen zur Befestigung am langen Ambossfortsatz bekannt. Die Ösen werden dabei auf den langen Ambossfortsatz aufgeschoben und an der gewünschten Stelle des Ambossfortsatzes fixiert. Dabei sind zum Aufweiten der Öse zur Erleichterung des Aufschiebens auf den Ambossfortsatz und/oder zur Fixierung der Prothese durch Zusammendrücken der Öse Werkzeuge erforderlich.

Zur Schaffung einer einfacher zu implantierenden Prothese schlägt die Erfindung eine Steigbügel-Prothese mit den Merkmalen des Anspruchs 1 vor. Hierdurch ist die Steigbügel-Prothese für den Operateur äußerst einfach zu handhaben. Der Clip wird einfach an der gewünschten Stelle des Ambossfortsatzes auf diesen aufgeschoben. Weitere Befestigungsmittel sind überflüssig. Die Prothese wird allein durch die Klemmkraft des elastischen Clips in Position gehalten. Der Clip ist in Form einer einseitig offenen Klammer ausgebildet und mit seiner Öffnung über den langen Ambossfortsatz aufschiebbar. Nach der Implantation umgreift der Clip den Ambossfortsatz mit Bereichen formschlüssig, wobei diese Bereiche durch einen bogenförmigen Abschnitt, der mit einigem Abstand zur Oberfläche des langen Ambossfortsatzes verläuft, miteinander verbunden sind. Hierdurch ist ein sicherer Halt der Prothese gewährleistet. Dabei ist es von Vorteil, dass der Clip den langen Ambossfortsatz nicht vollständig umgreift, sondern vorzugsweise nur an zwei sich gegenüberliegenden Bereichen. Wird der Clip dabei so angeordnet, dass Versorgungsgefäße des langen Ambossfortsatzes im Bereich des Bogens und der Öffnung zu liegen kommen, so kommt es im Gegensatz zu den bislang verwendeten Prothesen nicht zu einer Abschnürung der Gefäße für die Ernährung des Processus lenticularis. Durch die Ausgestaltung der Prothese ist außerdem die Gefahr der Nekrosenbildung auf ein Minimum reduziert.

Vorteilhafterweise kann zumindest der Clip aus Titan gefertigt sein. Die Restprothese kann beispielsweise auch aus Gold bestehen. Die Verwendung von Titan als Material für den Clip hat den Vorteil, dass dieser dadurch mit dem Ambossfortsatz verwachsen kann, wodurch die Prothese einen äußerst sicheren Halt erhält.

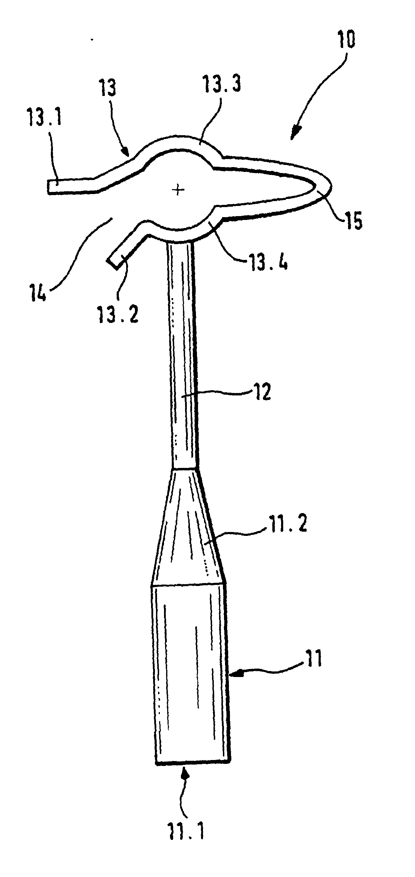

Nachfolgend wird ein bevorzugtes Ausführungsbeispiel einer erfindungsgemäßen Prothese anhand der Zeichnung näher erläutert.

Die einzige Figur zeigt eine Steigbügel-Prothese 10 in der Seitenansicht. Die Prothese 10 wird von einem kolbenförmigen Teil 11 gebildet, der mit seiner unteren Stirnseite 11.1 am ovalen Fenster anliegt und somit den Schall zum Innenohr überträgt. Der kolbenförmige Teil 11 weist nach oben hin eine Verjüngungszone 11.2 auf, an die sich ein Prothesenschaft 12 anschließt. Am oberen Ende des Schaftes 12 ist ein Clip 13 aus einem elastischen Material angeordnet. Der Clip 13 ist hierbei in Form einer einseitig offenen Klammer ausgebildet. Er kann mit seiner öffnung 14 über den langen Amboßfortsatz aufgeschoben werden. Dies wird durch die nach außen gebogenen Enden 13.1 und 13.2 des Clips 13 noch erleichtert. Nach der Implantation umgreift der Clip 13 mit zwei sich gegenüberliegenden Bereichen 13.3 und 13.4 den langen Amboßfortsatz formschlüssig. Damit werden die Versorgungsgefäße für den langen Amboßfortsatz nur in diesen beiden gegenüberliegenden Bereichen tangiert. Die übrigen Gefäße verlaufen im Bereich der öffnung 14 des Clips 13 sowie im Bereich eines bogenförmigen Abschnitts 15, der die beiden Bereiche 13.3 und 13.4 auf der der öffnung 14 gegenüberliegenden Seite 15 verbindet und mit einigem Abstand zur Oberfläche des langen Amboßfortsatzes verläuft. Auch im Bereich des Bogens 15 haben somit Versorgungsgefäße ausreichend Platz. Die dargestellte Prothese 10 ist somit nicht nur einfach zu handhaben, sondern sie beugt gleichzeitig Einschnürungen am langen Amboßfortsatz vor. Die Ernährung des Amboßfortsatzes wird somit nicht unterbrochen, wodurch dieser gesunderhalten bleibt. Der Clip 13 kann zweckmäßigerweise aus Titan gefertigt sein. Dadurch ist ein Verwachsen des Clips 13 mit dem Knochenmaterial des langen Amboßfortsatzes möglich.

## Patentansprüche

1. Steigbügel-Prothese zur Implantation im Mittelohr, wobei die Prothese (10) mit einem auf den langen Ambossfortsatz aufschiebbaren elastischen Clip (13) befestigbar ist und der Clip (13) in Form einer einseitig offenen Klammer ausgebildet und mit dieser Öffnung (14) an der gewünschten Stelle über den langen Ambossfortsatz aufschiebbar ist, **dadurch gekennzeichnet, dass** der Clip (13) den langen Ambossfortsatz nach der Implantation mit zwei Bereichen (13.3, 13.4) formschlüssig umgreift, wobei diese Bereiche (13.3, 13.4) durch einen bogenförmigen Abschnitt (15), der mit einigem Abstand zur Oberfläche des langen Ambossfortsatzes verläuft, miteinander verbunden sind.

2. Prothese nach Anspruch 1, **dadurch gekennzeichnet, dass** der Clip (13) den langen Ambossfortsatz an zwei sich gegenüberliegenden Bereichen (13.3, 13.4) formschlüssig umgreift.

3. Prothese nach Anspruch 1 oder 2, **dadurch gekennzeichnet, dass** nach der Implantation der Clip (13) so am langen Ambossfortsatz angeordnet ist, dass Versorgungsgefäße des langen Ambossfortsatzes im Bereich des Bogens (15) und der Öffnung (14) verlaufen.

4. Prothese nach einem der Ansprüche 1 bis 3, **dadurch gekennzeichnet, dass** mindestens der Clip (13) aus Titan gefertigt ist.

## Claims

1. Stapedial prosthesis for implantation in the middle ear, the prosthesis (10) being attachable with an elastic clip (13) that can be slipped on to the long incudal process and the clip (13) being configured with an opening (14) at one end that can be slipped over the long incudal process at the desired position, **characterized in that** after implantation the clip (13) positively clasps the long incudal process by two regions (13.3, 13.4), these regions (13.3, 13.4) being joined to each other by a bow portion (15) which extends some distance from the surface of the long incudal process.

2. Prosthesis according to Claim 1, **characterized in that** the clip (13) positively clasps the long incudal process in two opposite regions (13.3, 13.4).

3. Prosthesis according to Claim 1 or Claim 2, **characterized in that** after implantation the clip (13) is arranged on the long incudal process in such a way that [blood] supply vessels of the long incudal process extend in the region of the bow (15) and of the opening (14).

4. Prosthesis according to any one of Claims 1 to 3, **characterized in that** at least the clip (13) is made of titanium.

## Revendications

1. Prothèse d'étrier pour implantation dans l'oreille moyenne, la prothèse (10) étant fixée par une attache élastique (13) emmanchée sur le prolongement long de l'enclume, et l'attache (13) étant conçue sous la forme d'une agrafe ouverte d'un côté et, par l'intermédiaire de cette ouverture (14), étant emmanchée à l'endroit souhaité sur le prolongement long de l'enclume, **caractérisée en ce qu'**après l'implantation l'attache (13) entoure le prolongement long de l'enclume par complémentarité de formes avec deux zones (13.3, 13.4), ces zones (13.3, 13.4) étant reliées entre elles par une portion arquée (15) qui s'étend à une certaine distance de la surface du prolongement long de l'enclume.

2. Prothèse selon la revendication 1, **caractérisée en ce que** l'attache (13) entoure le prolongement long de l'enclume par complémentarité de formes dans deux zones (13.3, 13.4) situées en face l'une de l'autre.

3. Prothèse selon la revendication 1 ou 2, **caractérisée en ce qu'**après l'implantation l'attache (13) est disposée sur le prolongement long de l'enclume de façon que les vaisseaux d'irrigation du prolongement long de l'enclume s'étendent dans la zone de l'arc (15) et de l'ouverture (14).

4. Prothèse selon une des revendications 1 à 3, **caractérisée en ce qu'**au moins l'attache (13) est fabriquée en titane.
